# FASCICULE DE BREVET EUROPEEN

(11) **EP 0 508 909 B1**
(45) Date de publication et mention de la délivrance du brevet: **05.08.1998**
(21) Numéro de dépôt: 92420066.0
(22) Date de dépôt: 04.03.1992
(51) Int. Cl.: C12N 15/82, C12N 15/62, C12N 15/54, C12N 1/21, C12N 5/10, A01H 5/00

(54) **Gène chimère pour la transformation des plantes**
Chimäres Gen zur Transformation von Pflanzen
Chimeric gene for the transformation of plants

(30) Priorité: 05.03.1991 FR 9102872
(43) Date de publication de la demande: 14.10.1992
(60) Demande divisionnaire: 98102347.6
(73) Titulaire: RHONE-POULENC AGRO, 69009 Lyon (FR)
(72) Inventeur: Lebrun, Michel, F-69009 Lyon (FR); Leroux, Bernard, F-69380 Lozanne (FR); Sailland, Alain, F-69009 Lyon (FR)
(74) Mandataire: Chrétien, François

(56) Documents cités:
- EP-A- 0 218 571
- EP-A- 0 337 899
- WO-A-88/02402
- J. BIOL. CHEM. vol. 263, no. 29, 15 Octobre 1988, COMAI L.,ET AL.: 'Chloroplast transport of a ribulose biphosphate carboxylase small subunit-5-enolpyruvyl 3-phosphoshikimate synthase chimeric protein requires part of the mature small subunit in addition to the transit peptide'

## Description

La présente invention concerne de nouvelles séquences ADN de peptide de transit, de nouveaux gènes chimères et leur utilisation dans les plantes pour leur conférer une tolérance accrue à des herbicides en général et particulièrement ceux de la famille des phosphonométhylglycines.Elle concerne également les cellules végétales transformées à l'aide de ces gènes, les plantes transformées régénérées à partir de ces cellules ainsi que les plantes issues de croisements utilisant ces plantes transformées.

Le glyphosate ,le sulfosate ou la fosamétine sont des herbicides systémiques à large spectre de la famille des phosphonométhylglycines.Ils agissent essentiellement comme inhibiteurs compétitifs de la 5-enol pyruvylshikimate-3-phosphate synthase (EC 2.5.1.19) ou EPSPS vis à vis du PEP(phosphoenolpyruvate).Après leur application sur la plante,ils sont véhiculés dans la plante où ils s'accumulent dans les parties à croissance rapide, notamment les apex caulinaires et racinaires,provoquant l'altération jusqu'à la destruction des plantes sensibles.

L'EPSPS plastidiale, cible principale de ces produits est une enzyme de la voie de biosynthèse des acides aminés aromatiques,qui est codée par un ou des gènes nucléaires et synthétisée sous forme d'un précurseur cytoplasmique puis importée dans les plastes où elle s'accumule sous sa forme mature.

La tolérance des plantes au glyphosate et aux produits de la famille est obtenue par introduction stable dans leur génome d'un gène d'EPSPS d'origine végétale ou bactérienne mutée ou non quant aux caractéristiques d'inhibition par le glyphosate du produit de ce gêne.Etant donné le mode d'action du glyphosate et le degré de tolérance au glyphosate du produit des gènes utlisés, il est intéressant de pouvoir exprimer le produit de la traduction de ce gène de façon à permettre son accumulation importante dans les plastes.

Il est connu,par exemple d'après le brevet américain 4 535 060,de conférer à une plante une tolérance à un herbicide du type ci-dessus,en particulier la N-phosphonométhylglycine ou glyphosate, par introduction dans le génome des plantes d'un gène codant pour une EPSPS portant au moins une mutation rendant cette enzyme plus résistante à son inhibiteur compétitif(le glyphosate)après localisation de l'enzyme dans le compartiment plastidial (voire EP-A-0 189 707, EP-A-0 218 571 ou WO-A-88/02402).Ces techniques demandent cependant à être améliorées pour obtenir une plus grande fiabilité dans l'emploi de ces plantes en conditions agronomiques.
Dans la présente description,on entend par "plante" tout organisme multicellulaire différencié capable de photosynthèse et par "cellule végétale" toute cellule issue d'une plante et pouvant constituer des tissus indifférenciés tels que des cals ou des tissus différenciés tels que des embryons ou des parties de plantes des plantes ou des semences.

La présente invention a pour objet la production de plantes transformées ayant une tolérance accrue aux herbicides en général et particulièrement ceux de la famille des phosphonométhylglycines par régénération de cellules transformées à l'aide de nouveaux gènes chimères comportant un gène de tolérance à ces herbicides.L'invention concerne également ces nouveaux gènes chimères,les nouveaux peptides de transit qu'ils contiennent ainsi que les plantes les contenant rendues plus tolérantes par une accumulation de l'enzyme mutée, sous sa forme mature, dans les plantes.

Plus particulièrement l'invention a pour objet un gène chimère pour conférer aux plantes une tolérance accrue vis à vis d'un herbicide ayant pour cible l'EPSPS, comprenant, dans le sens de la transcription,une zone promotrice,une zone peptide de transit,une séquence d'un gène codant pour une enzyme de tolérance au glyphosate et une zone signal de polyadénylation non traduite en 3',caractérisé en ce que la zone peptide de transit comprend, dans le sens de la transcription, un premier peptide de transit issu de la PSU du gène de la ribulose-1,5-bis phosphate carboxylase oxygénase (RuBisCo), d'une plante une partie de séquence de la partie mature N terminale de la PSU du gène de la ribulose-1,5-bis phosphate carboxylase oxygénase(RuBisCo) d'une plante puis un second peptide de transit issu de la PSU du gène de la ribulose-1,5-bis phosphate carboxylase oxygénase (RuBisCo), d'une plante.

L'invention concerne également toute séquence ADN de la zone peptide de transit définie ci-dessus.

Les peptides de transit utilisables dans la zone de peptide de transit peuvent être en soi connus,d'origine végétale,par exemple issus de maïs, de tournesol, de pois, de tabac ou autres.Le premier et le second peptide de transit peuvent être identiques, analogues ou différents.Ils peuvent en outre comprendre chacun une ou plusieurs unités peptide de transit.

La partie de séquence de la partie mature N terminale est issue d'un gène végétal codant pour une enzyme à localisation plastidiale,comme par exemple un gène de maïs,de tournesol,de pois ou autres,l'espèce végétale d'origine pouvant être identique,analogue ou différente de celle dont sont issus respectivement le premier et le second peptide de transit.Par ailleurs la partie de séquence de la partie mature peut comprendre un nombre variable d'acides aminés,généralement de 10 à 40,de préférence de 18 à 33.

La construction de l'ensemble de la zone de transit peut être effectuée de manière en soi connue,notamment par fusion ou tout autre moyen convenable.Cette zone caractéristique a comme rôle de permettre le relargage d'une protéine mature et native avec une efficacité maximale.

La séquence codante pour la tolérance herbicide utilisable dans le gène chimère selon l'invention code pour une EPSPS mutée ayant un degré de tolérance au glyphosate.Cette séquence obtenue notamment par mutation du gène EPSPS peut être d'origine bactérienne, par exemple issu de Salmonella typhymurium (et nommée dans la suite "gène AroA"), ou végétale, par exemple de pétunia ou de tomate.Cette séquence peut comprendre une ou plusieurs mutations,par exemple la mutation Pro. 101 en Ser. ou encore les mutations Gly 96 en Ala.

La zone promotrice du gène chimère selon l'invention peut être composée avantageusement d'au moins un promoteur ou un fragment d'un promoteur d'un gène s'exprimant naturellement dans les plantes c'est à dire de promoteurs d'origine virale tels que celui de l'ARN 35S du virus de la mosaïque du choux (CaMV35S) ou d'origine végétale tels que la petite sous unité du gène de la ribulose-1,5-bisphosphate carboxylase(RubisCO) d'une culture comme le maïs ou le tournesol.

La zone signal de polyadénylation,non traduite en 3' du géne chimère selon l'invention peut être d'origine quelconque,par exemple bactérienne telle que celle du gène de nopaline synthase,ou végétale telle que celle de la petite sous unité de la RubisCO du maïs ou du tournesol.

Le gène chimère selon l'invention peut comprendre, en plus des parties essentielles ci-dessus, une zone intermédiaire non traduite(linker) entre la zone promotrice et la séquence codante et qui peut etre d'origine quelconque ,bactérienne,virale ou végétale.

### EXEMPLE:1 :CONSTRUCTION D'UN GENE CHIMERE:

On effectue la construction du gène chimère selon l'invention à partir des éléments suivants:

1) Promoteur" double CaMV" (c'est à dire dont une partie a été dupliquée):
le promoteur CaMV35S a été isolé par Odell et al(1985).Un clone,pJO 5-2 contenant environ 850bp en amont du site d'initiation de la transcription a été coupé par EcoRI-HindIII,les extrémités de ce fragment isolé ont été rendues franches par la polymérase de Klenow et le fragment inséré au site Hinc II du vecteur pUC19(Yannish-Perron et al ,1985).Ce promoteur a été digéré par ClaI,les extrémités remplies par la polymérase de Klenow,puis redigéré par HindIII.Un fragment HindIII-EcoRV,isolé à partir du même promoteur initial,a été introduit entre ces deux sites.Le promoteur ainsi obtenu possède une double zone amplificatrice en amont des éléments de régulation du promoteur CaMV35S.Il a été introduit sous forme d'un fragment HindIII-EcoRI dans le vecteur pRPA-BL 150 A alpha 2, décrit dans la demande de brevet français 88/04130, coupé par HindIII et EcoRI.

2)Zone de transit: les deux peptides de transit ainsi que les éléments de protéines matures utilisés proviennent de l'ADNc cloné de la petite sous unité du gène de la RubisCO de maïs,dont le gène a été décrit par Lebrun et al(1987), et de l'ADNc cloné de la petite sous unité du gène de la RubisCO de tournesol, isolé par Waksman et al(1987).Plus précisément,la zone de transit ,appelée peptide de transit optimisé, comprend,dans le sens de la traduction:
- un peptide de transit de la petite sous unité de la RuBisCO de tournesol,
- une séquence N terminale de 22 acides aminés de la partie mature de la petite sous unité de la RuBisCO de maïs,
- un peptide de transit de la petite sous unité de RuBisCO de maïs.

La construction utilisant ce peptide de transfert optimisé est nommée pRPA-BL 410.

D'autres séquences analogues peuvent être utilisées qui contiennent respectivement des séquences de 10 à 40 et de préférence 18 et 33 acides aminés.

Afin de fournir un élément comparatif,une autre construction a été effectuée avec un premier peptide de transit et la même partie de séquence mature mais sans second peptide de transit,selon l'art antérieur(pRPA-BL 294).

3) Gène de structure: il provient du gène muté à la position (Pro 101 en Ser) de l'EPSPS de Salmonella typhymurium isolé par Stalker et al(1985).Le clone pMG34-2(fourni par Calgene)a été linéarisé par XbaI puis traité à la nucléase de Vigna radiata.Après recoupure par SmaI les deux extrémités franches ont été ligaturées.Le clone obtenu possède un site NcoI sur l'ATG initiateur ainsi qu'un site SalI à 17pb en aval du codon stop.Ce clone a été nommé pRPA-BL 104.

4) Zone signal de polyadénylation: le fragment provient du gène de la nopaline synthase de pTi37 (Bevan et al,1983).Ce site est contenu dans un fragment MboI de 260pb(Fraley et al,1983;demande de brevet PCT 84/02913) qui a été traité par la polymérase de Klenow et cloné dans le site SmaI de M13 mp 18 pour introduire des sites BamHI et EcoRI respectivement aux extrémités 5' et 3.

Après coupure par BamHI et traitement à la nucléase de Vigna radiata puis coupure EcoRI et traitement par la polymerase de Klenow,le fragment résultant a ét introduit dans le vecteur p-BL 20(cf demande de brevet français 88/04130), coupé par XbaI et BamHI traités à la polymerase de Klenow. Après recoupe par SalI et SstI,on obtient un fragment d'environ 0,4kpb contenant la séquence nos 3' du côté du site Sal I et la bordure droite de l'ADN-T du côté du site SstI.

L'assemblage des différents éléments a été effectué de la façon suivante:

### Fusion" peptide de transit de la PSU de la RuBisCO de maïs/gène AroA":

Le peptide de transit de la PSU du gène de la RuBisCO de maïs provient d'un fragment EcoRI-SphI de 192pb; issu de l'ADNc correspondant au gène de la PSU du gène de la RuBisCO de maïs, décrit par Lebrun et al(1987),possédant un site NcoI recouvrant le codon initiateur de la traduction et un site SphI correspondant au site de clivage du peptide de transit.

Par traitement de l'extrémité SphI avec la polymerase du bactériophage T4 et en la ligaturant avec l'extrémité NcoI,traitée à la polymérase de Klenow,du gène AroA de pRPA-BL 104 recoupé EcoRI,on obtient la fusion traductionelle entre le peptide de transit de maïs et le gène de l'EPSPS bactérienne.

### Fusion peptide de transit de la PSU de la RuBisCO de maïs/séquence de 22 acides aminés de la partie mature de la PSU de la RuBisCO de maïs/ gène AroA:

De façon similaire un fragment EcoRI-HindII de 228bp de l'ADNc de la PSU du gène de la RubisCO de maïs est ligaturé avec l'extrémité NcoI traitée à la polymerase de Klenow du gène AroA de pRPA-BL 104 et recoupé par EcoRI.On obtient une fusion traductionnelle entre le peptide de transit de la PSU de la RuBisCO de maïs,les 22 acides aminés de la partie mature de la PSU de la RuBisCO de maïs et le gène de l'EPSPS bactérienne.

### Peptide de transit de la PSU de la RuBisCO de tournesol:

Le fragment est issu de l'ADNc isolé par Waksman et Freyssinet(1987).Un site SphI a été créé selon la méthode de Zoller et Smith(1984 au site de clivage du peptide de transit.Le peptide de transit de la PSU de la RuBisCO de tournesol ainsi obtenu est un fragment EcoRI-SphI de 171 pb.

### Fusion peptide de transit de la PSU de la RuBisCO de tournesol/séquence de 22 acides aminés de la partie mature de la PSU de la RuBisCO de maïs/ gène AroA:

La construction contenant la fusion peptide de transit de la PSU de la RuBisCO de maïs/séquence de 22 acides aminés de la PSU de la RuBisCO de maïs de la partie mature du gène de maïs a été coupée par EcoRI-SphI de 171 pb correspondant au peptide de transit de la PSU dedu gène de la RuBisCO de tournesol.Une construction résultante présente une substitution des fragments EcoRI-SphI et est une fusion traductionnelle "peptide de transit de la PSU de la RuBisCO de tournesol/séquence de 22 acides aminés de la partie mature de la PSU de la RuBisCO de maïs/gène AroA.

Le fragment EcoRI-SalI a été ligaturé avec le fragment SalI-SstI contenantla séquence nos 3' et la bordure droite de l'ADN-T.Le fragment EcoRI-SstI résultant comprenant"peptide de transit de la PSU de la RuBisCO de tournesol/séquence de 22 acides aminés de la partie mature de la PSU de la RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV.La fusion transcriptionelle "double CaMV/peptide de transit de la PSU de la RuBisCO de tournesol/séquence de 22 acides aminés de la partie mature de la PSU de la RuBisCO de maïs/gène AroA/nos 3' dans le vecteur 150 A alpha 2 a été nommé pRPA-BL 294.

### Fusion"peptide de transit de la PSU de la RuBisCO de tournesol/séquence de 22 acides aminés de la PSU de la RuBisCO de maïs/peptide de transit de la PSU de la RuBisCO de maïs/gène AroA":

La construction ci-dessus est coupée par NcoI-HindIII libérant le gène AroA.Puis elle est mise en ligation avec un fragment NcoI-Hind III de 1,5kpb contenant la fusion"peptide de transit de la PSU de la RuBisCO de maïs/gène AroA".Une construction résultante présente une substitution des fragments NcoI-HindIII et est une fusion traductionelle "peptide de transit de la PSU de la RuBisCO de tournesol/séquence de 22 acides aminés de la PSU de la RuBisCO de la partie mature du gène de maïs /peptide de transit de la PSU de la RuBisCO de maïs/gène AroA".

Le fragment EcoRI-SalI a été ligaturé avec le fragment SalI-SstI contenantla séquence nos 3' et la bordure droite de l'ADN-T.Le fragment EcoRI-SstI résultant comprenant"peptide de transit de la PSU de la RuBisCO de tournesol/séquence de 22 acides aminés de la PSU de la RuBisCO de la partie mature du gène de maïs /peptide de transit de la PSU de la RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV.La fusion transcriptionelle "double CaMV/peptide de transit de la PSU de la RuBisCO de tournesol/séquence de 22 acides aminés de la PSU de la RuBisCO de la partie mature du gène de maïs/peptide de transit de la PSU de la RuBisCO de maïs/gène AroA/nos 3' dans le vecteur 150 A alpha 2 a été nommé pRPA-BL 410.

### EXEMPLE 2:RESISTANCE DES PLANTES TRANSFORMEES

### 1°Transformation:

Le vecteur est introduit dans la souche non oncogène d'Agrobacterium EHA 101(Hood et al,1987)porteuse du cosmide pTVK 291(Komari et al,1986).La technique de transformation est basée sur la procédure de Horsh et al(1985).

### 2° Régénération:

La régénération du tabac PBD6(provenance SEITA France) à partir d'explants foliaires est réalisée sur un milieu de base Murashige et Skoog(MS) comprenant 30g/l de saccharose ainsi que 200ug/ml de kanamycine.Les explants foliaires sont prélevés sur des plants en serre ou in vitro et transformés selon la technique des disques foliaires(Science 1985,Vol 227,p.1229-1231) en trois étapes successives:la première comprend l'induction des pousses sur un milieu MS additionné de 30g/l de saccharose contenant 0.05mg/l d'acide naphtylacétique (ANA) et 2 mg/l de benzylaminopurine (BAP) pendant 15 jours.Les pousses formées au cours de cette étape sont ensuite développées par culture sur un milieu MS additionné de 30 g/l de saccharose mais ne contenant pas d'hormone,pendant 10 jours.Puis on prélève des pousses développées et on les cultive sur un milieu d'enracinement MS à teneur moitié en sels, vitamines et sucres et ne contenant pas d'hormone.Au bout d'environ 15 jours,les pousses enracinées sont passées en terre.

### 3°Mesure de la tolérance au glyphosate:

a)in vitro:la tolérance est mesurée par pesée de la masse de cals extrapolée pour 100 disques foliaires de 0,5 cm de diamètre, au bout de 30jours de croissance, sur un milieu MS additionné de 30g/l de sucrose,de 0,05mg/l d'acide naphtalène acétique et de 2mg/lde BAP contenant 35 ppm de glyphosate et 200 microg/ml de kanamycine.Dans ces conditions on observe que, pour les plants de tabac modifiés par le gène chimère de pRPA BL 410selon l'invention, la masse de cals est de 34g,alors que pour les plants modifiés par le gène chimère sans second peptide de transit la masse n'est que de 12g.
b)in vivo:30 plants issus de la régénération des tabacs transformés respectivement par pRPA-BL 294 et pRPA-BL 410 sont passés en serre et traités au stade 5 feuilles par pulvérisation avec une suspension aqueuse à une dose correspondant à 0,6kg/ha de glyphosate(Round up).Au bout de 21 jours on effectue une observation phénotypique des plants par rapport à des plants témoins non transformés. Dans ces conditions on constate que les plantes transformées par pRPA-BL 410 présentent une phytotoxicité négligeable alors que les plants témoins sont complètement détruits;de plus les plants transformés avec un gène chimère différent du précédent par l'absence de second peptide de transit présentent une phytotoxicité au moins égale à 30% de destruction.

Ces résultats montrent clairement l'amélioration apportéé par utilisation d'un gène chimère selon l'invention pour un même gène codant pour la tolérance au glyphosate.

Les plantes transformées selon l'invention peuvent être utilisées comme parents pour l'obtention de lignées et d'hybrides ayant une tolérance accrue au glyphosate

### Exemple 3 :

Des colzas de printemps, cultivar Westar, résistants au glyphosate, ont été obtenus en utilisant la technique de BOULTER et al., 1990 (Plant Science, 70 : 91-99), avec la pRPA-BL 410. Ces plantes ont résisté à un traitement en serre au glyphosate à 400 g m.a./ha, traitement qui détruit les plantes non transgéniques.

## Revendications

1. Séquence d'ADN codant pour un peptide de transit caractérisée en ce qu'elle comprend, dans le sens de la transcription,
- une séquence codant pour un premier peptide de transit issu de la petite sous unité d'un gène de Ribulose-1,5-biphosphate carboxylase/oxygénase (RuBisCO) d'une plante,
- une partie de séquence de la partie mature N terminale de la petite sous unité du gène de RuBisCO d'une plante,
- puis une séquence codant pour un second peptide de transit issu de la petite sous unité d'un gène de RuBisCO d'une plante.

2. Séquence selon la revendication 1,caractérisée en ce que la séquence codant pour le second peptide de transit est issue de la même plante que la séquence codant pour le premier peptide de transit.

3. Séquence selon la revendication 1, caractérisée en ce que la séquence codant pour le second peptide de transit est issu d'une plante différente de celle du premier peptide de transit.

4. Séquence selon l'une des revendications 1 à 3 caractérisée en ce qu'au moins une séquence codant pour un des peptides de transit est issue du même gène que celui d'origine de la séquence de la partie mature.

5. Séquence selon l'une des revendications 1 à 4 caractérisée en ce qu'au moins une séquence codant pour un des peptides de transit est issu de la petite sous unité du gène de RuBisCO du maïs.

6. Séquence selon l'une des revendications 1 à 5, caractérisée en ce qu'au moins une séquence codant pour un des peptides de transit est issu de la petite sous unité du gène de RuBisCO du tournesol.

7. Séquence selon l'une des revendications 1 à 6, caractérisée en ce que la séquence de la partie mature est issue de la petite sous unité du gène de RuBisCO de maïs.

8. Séquence selon l'une des revendications 1 à 6, caractérisée en ce que la séquence de la partie mature est issue de la petite sous unité du gène de RuBisCO de tournesol.

9. Séquence selon la revendication 8, caractérisée en ce que la séquence de la partie mature comprend de 10 à 40 acides aminés.

10. Séquence selon l'une des revendications 1 à 9, caractérisée en ce que la séquence de la partie mature comprend de 18 à 33 acides aminés.

11. Séquence d'ADN codant pour un peptide de transit optimisé, caractérisé en ce qu'il comprend, dans le sens de la traduction:
- un peptide de transit de la petite sous unité de la RuBisCO de tournesol,
- une séquence N terminale de 22 acides aminés de la partie mature de la petite sous unité de la RuBisCO de maïs,
- un peptide de transit de la petite sous unité de RuBisCO de maïs.

12. Gène chimère pour conférer aux plantes une tolérance accrue vis à vis d'un herbicide ayant pour cible la 5-enol pyruvylshikimate-3-phosphate synthase (EPSPS), comprenant, dans le sens de la transcription,une zone promotrice, une zone peptide de transit, une séquence codante pour la tolérance au glyphosate et une zone signal de polyadénylation non traduite en 3', caractérisé en ce que la zone peptide de transit comprend une séquence d'ADN selon l'une des revendications 1 à 11.

13. Gène chimère selon la revendication 12, caractérisé en ce que l'herbicide est le glyphosate.

14. Gène chimère selon l'une des revendications 12 ou 13, caractérisé en ce que la séquence codante de la tolérance au glyphosate est d'origine bactérienne.

15. Gène chimère selon l'une des revendications 12 ou 13, caractérisé en ce que la séquence codante de la tolérance au glyphosate est d'origine végétale.

16. Vecteur pour la transformation des plantes, caractérisé en ce qu'il comprend un gène chimère selon l'une des revendications 12 à 15.

17. *Agrobacterium sp.* caractérisée en ce qu'elle contient un vecteur selon la.revendication 16.

18. Cellule végétale transformée, caractérisée en ce qu'elle contient un gène chimère selon l'une des revendications 12 à 15.

19. Procédé de construction d'un gène chimère selon l'une des revendications 12 à 15, caractérisé en ce qu'on isole respectivement au moins deux zones de peptide de transit et au moins une séquence de partie mature de gènes végétaux plastidiaux appropriés, ainsi qu'au moins une séquence codant pour la tolérance au glyphosate et une zone signal de polyadénylation appropriée et que ensuite on les assemble dans le sens de la transcription du gène de tolérance.

## Patentansprüche

1. DNA-Sequenz, die ein Transit-Peptid codiert, dadurch gekennzeichnet, daß
sie in Transkriptionsrichtung aufweist:
- eine Sequenz, die ein erstes Transit-Peptid codiert und aus der kleinen Untereinheit eines Gens für Ribulose-1,5-biphosphat-carboxylase/oxygenase (RuBisCO) einer Pflanze stammt,
- eine Teilsequenz des N-terminalen, reifen Teils der kleinen Untereinheit des RuBisCO-Gens einer Pflanze,
- eine Sequenz, die ein zweites Transit-Peptid codiert und aus der kleinen Untereinheit eines RuBisCO-Gens einer Pflanze stammt.

2. Sequenz nach Anspruch 1,
dadurch gekennzeichnet, daß
die Sequenz, die das zweite Transit-Peptid codiert, aus der gleichen Pflanze stammt wie die Sequenz, die das erste Transit-Peptid codiert.

3. Sequenz nach Anspruch 1,
dadurch gekennzeichnet, daß
die Sequenz, die das zweite Transit-Peptid codiert, aus einer Pflanze stammt, die von der Pflanze des ersten Transit-Peptids verschieden ist.

4. Sequenz nach einem der Ansprüche 1 bis 3,
dadurch gekennzeichnet, daß
mindestens eine Sequenz, die eines der Transit-Peptide codiert, aus dem Gen stammt, das mit dem Ursprungsgen der Sequenz des reifen Teils identisch ist.

5. Sequenz nach einem der Ansprüche 1 bis 4,
dadurch gekennzeichnet, daß
mindestens eine Sequenz, die eines der Transit-Peptide codiert, aus der kleinen Untereinheit des RuBisCO-Gens von Mais scammt.

6. Sequenz nach einem der Ansprüche 1 bis 5,
dadurch gekennzeichnet, daß
mindestens eine Sequenz, die eines der Transit-Peptide codiert, aus der kleinen Untereinheit des RuBisCO-Gens von Sonnenblumen stammt.

7. Sequenz nach einem der Ansprüche 1 bis 6,
dadurch gekennzeichnet, daß
die Sequenz des reifen Teils aus der kleinen Untereinheit des RuBisCO-Gens von Mais stammt.

8. Sequenz nach einem der Ansprüche 1 bis 6,
dadurch gekennzeichnet, daß
die Sequenz des reifen Teils aus der kleinen Untereinheit des RuBisCO-Gens von Sonnenblumen stammt.

9. Sequenz nach Anspruch 8,
dadurch gekennzeichnet, daß
die Sequenz des reifen Teils 10 bis 40 Aminosäuren umfaßt.

10. Sequenz nach einem der Ansprüche 1 bis 9,
dadurch gekennzeichnet, daß
die Sequenz des reifen Teils 18 bis 33 Aminosäuren umfaßt.

11. DNA-Sequenz, die ein optimiertes Transit-Peptid codiert, dadurch gekennzeichnet, daß
sie in Translationsrichtung aufweist:
- ein Transitpeptid der kleinen Untereinheit von RuBisCO aus Sonnenblumen,
- eine N-terminale Sequenz von 22 Aminosäuren des reifen Teils der kleinen Untereinheit von RuBisCO aus Mais,
- ein Transit-Peptid der kleinen Untereinheit von RuBisCO aus Mais.

12. Chimäres Gen, um Pflanzen eine größere Toleranz gegenüber Herbiziden zu verleihen, die auf die 5-Enolpyruvylshikimat-3-phosphat-synthase (EPSPS) einwirken, das in Transkriptionsrichtung folgende Bereiche aufweist: einen Promotorbereich, einen Transit-Peptid-Bereich, eine Sequenz, die die Toleranz gegenüber Glyphosat codiert und einen nicht translatierten Signalbereich der Polyadenylierung in 3'-Stellung,
dadurch gekennzeichnet, daß
der Bereich des Transit-Peptids eine DNA-Sequenz nach einem der Ansprüche 1 bis 11 umfaßt.

13. Chimäres Gen nach Anspruch 12,
dadurch gekennzeichnet, daß
das Herbizid das Glyphosat ist.

14. Chimäres Gen nach einem der Ansprüche 12 oder 13,
dadurch gekennzeichnet, daß
die Sequenz, die die Toleranz gegenüber Glyphosat codiert, bakteriellen Ursprungs ist.

15. Chimäres Gen nach einem der Ansprüche 12 oder 13,
dadurch gekennzeichnet, daß
die Sequenz, die die Toleranz gegenüber Glyphosat codiert, pflanzlichen Ursprungs ist.

16. Vektor zur Transformation von Pflanzen,
dadurch gekennzeichnet, daß
er ein chimäres Gen nach einem der Ansprüche 12 bis 15 aufweist.

17. *Agrobacterium sp,*
dadurch gekennzeichnet, daß
es einen Vektor nach Anspruch 16 enthält.

18. Transformierte Pflanzenzelle,
dadurch gekennzeichnet, daß
sie ein chimäres Gen nach einem der Ansprüche 12 bis 15 enthält.

19. Verfahren zur Konstruktion eines chimären Gens nach einem der Ansprüche 12 bis 15,
dadurch gekennzeichnet, daß
mindestens zwei Transit-Peptid-Bereiche und mindestens eine Sequenz des reifen Teils geeigneter pflanzlicher Plastidengene sowie mindestens eine Sequenz, die die Glyphosat-Toleranz codiert, und ein geeigneter Signalbereich der Polyadenylierung isoliert werden und daß dann die verschiedenen Bereiche in Transkriptionsrichtung des Toleranzgens zusammengefügt werden.

## Claims

1. DNA sequence encoding a transit peptide, characterized in that it comprises, in the direction of transcription,
- a sequence encoding a first transit peptide derived from the small subunit of a RuBisCO gene of a plant,
- a partial sequence of the N-terminal mature part of the small subunit of the RuBisCO gene of a plant,
- and then a sequence encoding a second peptide derived from the small subunit of a RuBisCO gene of a plant.

2. Sequence according to Claim 1, characterized in that the sequence encoding the second transit peptide is derived from the same plant as the sequence encoding the first transit peptide.

3. Sequence according to Claim 1, characterized in that the sequence encoding the second transit peptide is derived from a plant which is different from that for the first transit peptide.

4. Sequence according to one of Claims 1 to 3, characterized in that at least one sequence encoding one of the transit peptides is derived from the same gene as the original one for the sequence of the mature part.

5. Sequence according to one of Claims 1 to 4, characterized in that at least one sequence encoding one of the transit peptides is derived from the small subunit of the gene for maize RuBisCO.

6. Sequence according to one of Claims 1 to 5, characterized in that at least one sequence encoding one of the transit peptides is derived from the small subunit of the gene for sunflower RuBisCO.

7. Sequence according to one of Claims 1 to 6, characterized in that the sequence of the mature part is derived from the small subunit of the gene for maize RuBisCO.

8. Sequence according to one of Claims 1 to 6, characterized in that the sequence of the mature part is derived from the small subunit of the gene for sunflower RuBisCO.

9. Sequence according to Claim 8, characterized in that the sequence of the mature part comprises from 10 to 40 amino acids.

10. Sequence according to one of Claims 1 to 9, characterized in that the sequence of the mature part comprises from 18 to 33 amino acids.

11. DNA sequence encoding an optimized transit peptide, characterized in that it comprises, in the direction of translation:
- a transit peptide for the small subunit of sunflower RuBisCO,
- an N-terminal sequence of 22 amino acids for the mature part of the small subunit of maize RuBisCO,
- a transit peptide for the small subunit of maize RuBisCO.

12. Chimeric gene for conferring on plants an increased tolerance to a herbicide having as its target 5-(enolpyruvyl)shikimate-3-phosphate synthase (EPSPS), comprising, in the direction of transcription, a promoter region, a transit peptide region, a coding sequence for glyphosate tolerance and an untranslated polyadenylation signal region in 3', characterized in that the transit peptide region comprises a DNA sequence according to one of Claims 1 to 11.

13. Chimeric gene according to Claim 12, characterized in that the herbicide is glyphosate.

14. Chimeric gene according to either of Claims 12 and 13, characterized in that the coding sequence for glyphosate tolerance is of bacterial origin.

15. Chimeric gene according to either of Claims 12 and 13, characterized in that the coding sequence for glyphosate tolerance is of plant origin.

16. Vector for transforming plants, characterized in that it comprises a chimeric gene according to one of Claims 12 to 15.

17. *Agrobacterium sp.*, characterized in that it contains a vector according to Claim 16.

18. Transformed plant cell, characterized in that it contains a chimeric gene according to one of Claims 12 to 15.

19. Process for constructing a chimeric gene according to one of Claims 12 to 15, characterized in that at least two transit peptide regions and at least one sequence of the mature part of suitable plastid-localized plant genes as well as at least one sequence encoding glyphosate tolerance and a suitable polyadenylation signal region are isolated respectively, and in that they are then assembled in the direction of transcription of the tolerance gene.
